# EUROPÄISCHE PATENTSCHRIFT

(11) **EP 2 760 444 B1**
(45) Veröffentlichungstag und Bekanntmachung des Hinweises auf die Patenterteilung: **22.04.2020**
(21) Anmeldenummer: 12778040.1
(22) Anmeldetag: 01.10.2012
(51) Int. Cl.: C07C 65/19, C07C 51/41, C07C 51/42, C07C 51/493, C07D 311/80

(54) **CANNABINOIDCARBONSÄUREN, SALZE VON CANNABINOIDCARBONSÄUREN, DEREN HERSTELLUNG UND ANWENDUNGEN**
CANNABINOID CARBOXYLIC ACIDS, SALTS OF CANNABINOID CARBOXYLIC ACIDS, AND THE PRODUCTION AND USES OF SAME
ACIDES CARBOXYLIQUES DE CANNABINOÏDE, SELS D'ACIDES CARBOXYLIQUES DE CANNABINOÏDE, ET FABRICATION ET UTILISATION DESDITS ACIDES ET SELS D'ACIDES CARBOXYLIQUES DE CANNABINOÏDE

(30) Priorität: 29.09.2011 DE 102011114528
(43) Veröffentlichungstag der Anmeldung: 06.08.2014
(73) Patentinhaber: THC Pharm GmbH The Health Concept, 60599 Frankfurt am Main (DE)
(72) Erfinder: HERKENROTH, Thomas, 83098 Brannenburg (DE); STEUP, Christian, 65719 Hofheim am Taunus (DE)
(74) Vertreter: Vossius, Corinna
(86) Internationale Anmeldenummer: PCT/EP2012/004107
(87) Internationale Veröffentlichungsnummer: WO 2013/045115

(56) Entgegenhaltungen:
- EP-A1- 1 559 423
- EP-A1- 2 314 580
- WO-A1-94/14790
- WO-A2-03/064407
- DE-B4- 10 106 024
- CROMBIE ET AL.: J. CHEM. RESEARCH, 1977, Seiten 1301-1345, XP002690047, in der Anmeldung erwähnt
- R. MECHOULAM ET AL.: TETRAHEDRON, Bd. 21, 1965, Seite 1223, XP002690048, in der Anmeldung erwähnt
- R. MECHOULAM ET AL.: "Carboxylation of recorcinols with methylmagnesium carbonate. Synthesis of cannabinoid acids.", CHEMICAL COMMUNICATIONS, 1969, Seiten 343-344, XP002690049,

## Beschreibung

### Technisches Gebiet

Die Erfindung betrifft allgemein Salze von Cannabinoidcarbonsäuren, Verfahren zu deren Herstellung sowie deren Anwendungen.

### Stand der Technik

Cannabinoide kommen in der Hanfpflanze Cannabis sativa in Form ihrer Carboxylderivate, den Cannabinoidcarbonsäuren vor, aus denen die sogenannten "Neutralcannabinoide" durch Decarboxylierung, d.h., Abspaltung von CO₂, hervorgehen. So entsteht bspw. Cannabidiol (CBD - (I)) durch Decarboxylierung von Cannabidiolsäure (CBDS - (II)). 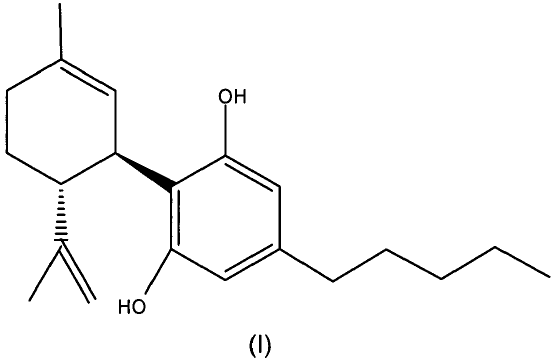 (-)-CBD

2-((1R,6R)-3-Methyl-6-(prop-1-en-2-yl)cyclohex-2-enyl)-5-pentylbenzen-1,3-diol 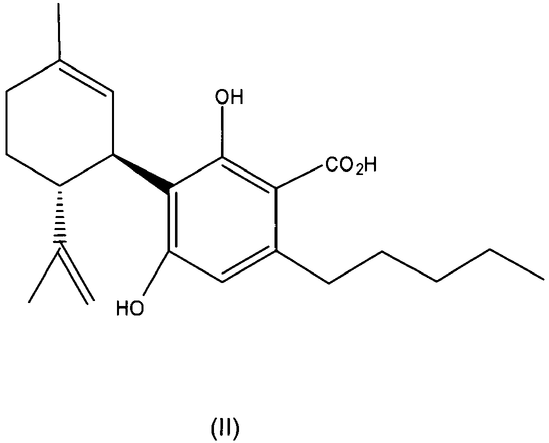 Cannabidiolsäure (CBDS)

2,4-Dihydroxy-3-((1R,6R)-3-methyl-6-(prop-1-en-2-yl)cyclohex-2-enyl)-6-pentylbenzoesäure

Δ⁹-Tetrahydrocannabinol (Δ⁹-THC-Dronabinol (III)) entsteht durch Decarboxylierung aus den stellungsisomeren Δ⁹-Tetrahydrocannabinolsäuren, der Δ⁹-Tetrahydrocannabinolsäure-A (THCS-A - (IV)) und der Δ⁹-Tetrahydrocannabinolsäure-B (THCS-B - (V)). 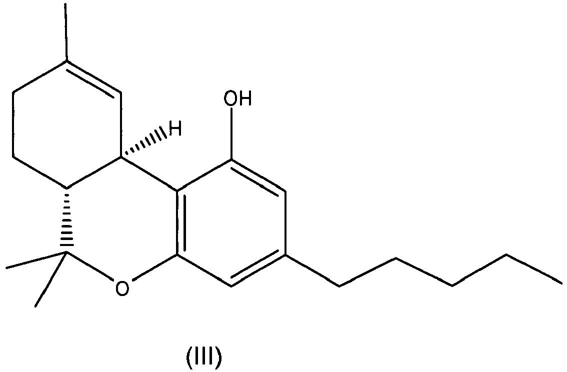 (-)-Δ⁹-THC

(6aR,10aR)-6,6,9-Trimethyl-3-pentyl-6a,7,8,10a-tetrahydro-6H-benzo[c]chromen-1-ol 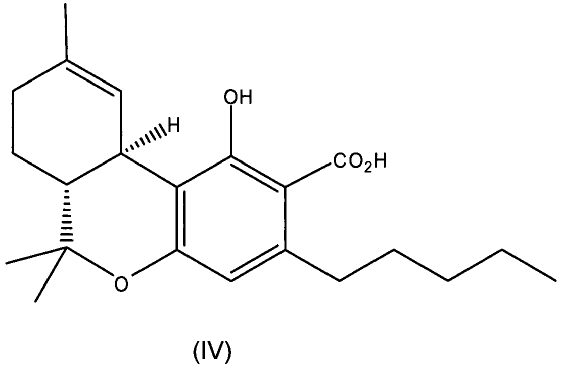 Δ⁹-Tetrahydrocannabinolsäure-A (THCS-A)

(6aR,10aR)-1-Hydroxy-6,6,9-trimethyl-3-pentyl-6a,7,8,10a-tetrahydro-6H-benzo[c]chromen-2-carbonsäure, 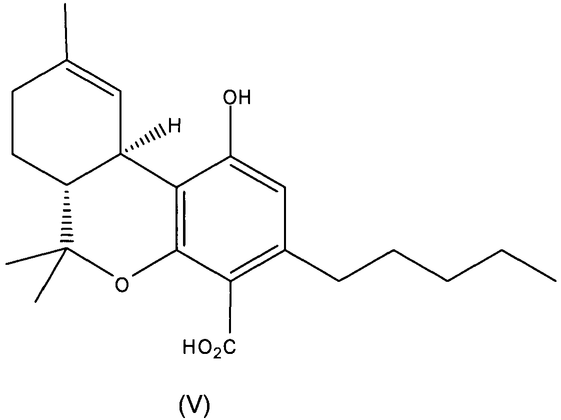 Δ⁹-Tetrahydrocannabinolsäure-B (THCS-B)

(6aR,10aR)-1-Hydroxy-6,6,9-trimethyl-3-pentyl-6a,7,8,10a-tetrahydro-6H-benzo[c]chromen-4-carbonsäure)

Cannabigerol (VI) entsteht auf diese Weise aus Cannabigerolsäure (CBGS - (VII)). 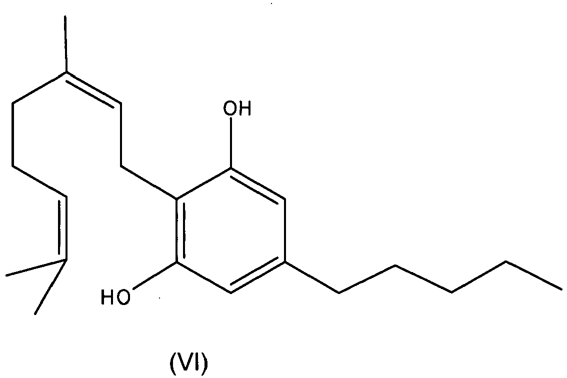 Cannabigerol (CBG)
(Z)-2-(3,7-Dimethylocta-2,6-dienyl)-5-pentylbenzen-1,3-diol 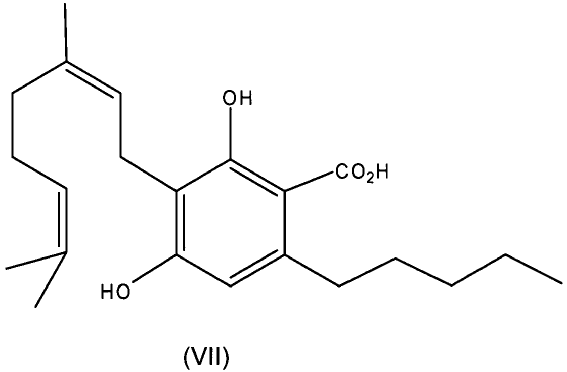 Cannabigerolsäure (CBGS)

(Z)-3-(3,7-Dimethylocta-2,6-dienyl)-2,4-dihydroxy-6-pentylbenzoesäure

Analoges gilt auch für die natürlich vorkommenden Spurencannabinoide wie z.B. Δ⁸-Tetrahydrocannabinol, Cannabicyclol, Cannabicitran, Cannabielsoin oder den Homologen der erwähnten Cannabinoide. Dabei sind im Falle eines geschlossenen Pyranrings wie beim Δ⁹-THC (III) jeweils zwei stellungsisomere Carbonsäuren -A und -B möglich. In der Natur kommen beide Formen vor (vgl. z.B. R. Mechoulam et al., Tetrahedron, 1965, 21, 1223; F. Korte et al., Angew. Chem. Internatl. Ed., 1965, 4, 872 und R. Mechoulam et al., Tetrahedron Letters, 1969, 2339).

Bisher mussten Verbindungen wie Dronabinol (III) und seine Homologen durch aufwändige Verfahren wie präparative Chromatographie gereinigt werden. So offenbart die US-4,025,516 ein Verfahren zur Herstellung von Dronabinol durch Kondensation von (+)-p-Mentha-2,8-dien-1-ol mit Olivetol in Gegenwart von BF₃-Etherat. Aus der US-5,342,971 ist die Synthese von Dronabinol aus Cannabidiolsäureestern in Gegenwart von Lewis-Säuren und anschließender Hydrolyse bekannt. Die Herstellung von Dronabinol aus Tetrahydrocannabinol-reichem Cannabis mit anschließender Destillation und/oder Chromatographie ist in der Internationalen Patentanmeldung WO 00/25127 beschrieben. Schließlich offenbart die DE 101 06 024 B4 ein Verfahren zur Herstellung von Dronabinol, bei dem a) aus Pflanzenmaterialien Cannabidiol und/oder Cannbidiolsäure isoliert wird, b) das gegebebenfalls durch Decarboxylierung gewonnene Cannabidiol in einem organischen oder nicht polaren Lösungsmittel in Gegenwart von Lewis-Katalysatoren zu Dronabinol zyklisiert wird, c) dieses durch ein chromatographisches Verfahren isoliert und d) der aus dem Eluat nach Abdestillation des Lösungsmittels gewonnene Rückstand durch Vakuumdestillation gereinigt wird.

Die Herstellung kristallisierbarer Vorstufen wie der Ester (z.B. des 3,5-Dinitrobenzoylesters), deren Umkristallisation und die nachfolgende Verseifung sind relativ aufwändig und außerdem mit der Gefahr verbunden, weitere Verunreinigungen zu erzeugen.

Die Reaktion, die aus den oben genannten Cannabinoidcarbonsäuren oder deren Salzen die Neutralcannabinoide entstehen lässt, verläuft bei Raumtemperatur in der Regel langsam, kann aber durch Erwärmen und/oder Zusatz von Katalysatoren so beschleunigt werden, dass der CO₂-Verlust innerhalb weniger Minuten oder praktisch unverzüglich verläuft. Diese Reaktion kann so gesteuert werden, dass sie praktisch quantitativ und ohne die Bildung weiterer Nebenprodukte verläuft.

Aus dem Gesagten ergibt sich, dass reine Cannabinoidcarbonsäuren praktisch ideale Vorläufer darstellen, um daraus reine Neutralcannabinoide zu gewinnen. Es wäre daher für die Herstellung reiner Cannabinoide ein wesentlicher Fortschritt, ein Verfahren bereit zu stellen zu können, das es erlaubt, auf wirtschaftliche Weise Cannabinoidcarbonsäuren zu gewinnen und zu reinigen.

EP 1 559 423 A1 offenbart Cannabinoidcarbonsäuren, insbesondere zur Behandlung von Hauterkrankungen.

EP 2 314580A1 beschreibt ein Verfahren zur Herstellung von Cannabinoiden durch Verseifung und Decarboxylierung von Cannabinoidcarbonsäureestern, wobei ein Kaliumsalz der Cannabinoidcarbonsäure verwendet wird.

WO 03/064407 A2 offenbart Alkali- und Erdalkalisalze der Tetrahydrocannabinolsäure, wobei die daraus erhaltene Säure thermisch zersetzt wird.

Der Erfindung liegt daher die Aufgabe zugrunde, ein Verfahren zur Herstellung von möglichst reinen Salzen gemäß Anspruch 1, vorzugsweise kristallinen Salzen natürlicher oder synthetischer Cannabinoidcarbonsäuren bereit zu stellen, aus denen auf einfachem Wege reine Neutralcannabinoide erhalten werden können.

Eine weitere Aufgabe besteht darin, ein solches Verfahren bereit zu stellen, das mit relativ geringem Aufwand durchgeführt werden kann und weniger anfällig für die Bildung von Verunreinigungen ist.

### Offenbarung der Erfindung

Die vorliegende Erfindung löst diese Aufgabe durch Bereitstellen eines Verfahrens zur Herstellung kristalliner und löslicher Salze von Cannabinoidcarbonsäuren gemäß Anspruch 1 durch Herstellen von synthetischen Cannabinoidcarbonsäuren in einer chemischen Reaktion oder Extraktion von natürlichen Cannabinoidcarbonsäuren aus Pflanzenmaterialien oder Zellkulturen von Cannabis sativa und anschließendem Versetzen mit einer geeigneten organischen Base, anorganischen Base und/oder einem geeigneten anorganischen oder organischen Salz in einem geeigneten Lösungsmittel.

Die vorliegende Erfindung löst diese Aufgabe durch Bereitstellen eines Verfahrens zur Herstellung amorpher und kristalliner Salze, insbesondere reiner, vorzugsweise kristalliner Salze von Cannabinoidcarbonsäuren durch Herstellen von synthetischen Cannabinoidcarbonsäuren in einer chemischen Reaktion oder Extraktion von natürlichen Cannabinoidcarbonsäuren aus Pflanzenmaterialien oder Zellkulturen von Cannabis sativa und anschließendem Versetzen mit einer geeigneten organischen Base, anorganischen Base und/oder einem geeigneten anorganischen oder organischen Salz in einem geeigneten Lösungsmittel. Als rein werden hier Stoffmischungen mit einem Salzanteil von >90 Gew.-% verstanden.

Die vorliegende Erfindung beinhaltet die Herstellung natürlicher oder synthetischer Cannabinoidcarbonsäuren und deren Salzen (1) bis (4), umfassend alle Stereoisomeren sowie deren Mischungen, insbesondere die erstmalige Herstellung kristalliner Salze von Cannabinoidcarbonsäuren, ihre Reinigung durch Umkristallisation und die Gewinnung reiner Neutralcannabinoide daraus.

Ferner werden Verfahren offenbart, mittels denen aus Cannabinoidcarbonsäuren amorphe oder kristalline Salze gewonnen werden können bzw. amorphe oder gelöste Salze von Cannabinoidcarbonsäuren in kristalline überführt werden können und mittels denen aus reinen Cannabinoidcarbonsäuren bzw. deren Salzen bzw. deren Lösungen reine Neutralcannabinoide hergestellt werden können.

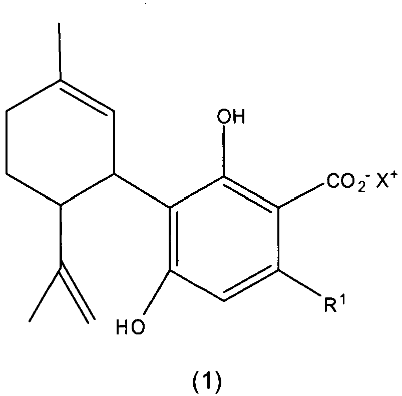

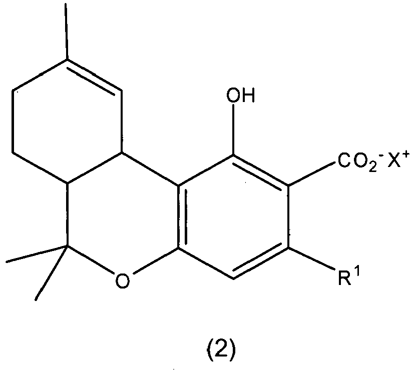

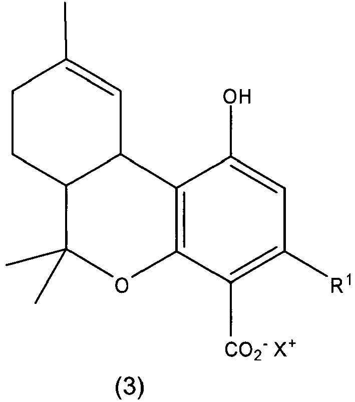

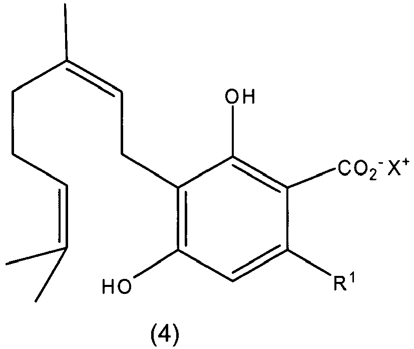

Dabei ist R¹ ein geradkettiger, verzweigter oder zyklischer Kohlenwasserstoffrest mit einem C-Atom bis zu 12 C-Atomen.

X⁺ ist bevorzugt ausgewählt ist aus der Gruppe bestehend aus NH₄⁺, und primären, sekundären, tertiären oder quartären organischen Ammoniumionen mit bis zu 48 C-Atomen, welche noch weitere funktionelle Gruppen tragen können.

Beispiele für mehrwertige Ammoniumionen sind N,N-Dicyclohexylamin-H⁺ und N,N-Dicyclohexyl-N-ethylamin-H⁺. X⁺ kann auch das Hydrogenium-Kation eines pharmazeutischen Wirkstoffes mit mindestens einem basischen Stickstoffatom sein, wie z.B. Morphin, Methadon (bzw. eines Enantiomeren dessen) oder Hydromorphon.

Die Herstellung reiner, vorzugsweise kristalliner Salze von Cannabinoidcarbonsäuren umfasst zwei Schritte: In einem ersten Schritt werden synthetische Cannabinoidcarbonsäuren in einer chemischen Reaktion hergestellt oder natürliche Cannabinoidcarbonsäuren aus Pflanzenmaterial oder Zellkulturen von Cannabis sativa extrahiert.

In einem zweiten Schritt werden die so hergestellten Cannabinoidcarbonsäuren oder Cannabinoidcarbonsäuren-reichen Extrakte in einem geeigneten Lösungsmittel mit einer geeigneten organischen Base, einer geeigneten anorganischen Base und/oder einem geeigneten anorganischen oder organischen Salz versetzt, so dass die schwerlöslichen Salze der Cannabinoidcarbonsäuren ausfallen. Diese können z.B. durch Filtration abgetrennt und wenn nötig durch Umkristallisation gereinigt werden.

In einem weiteren Schritt lassen sich aus den so hergestellten reinen, vorzugsweise kristallinen Salzen reine Cannabinoide gewinnen.

Dies kann geschehen durch Austreiben der Cannabinoidcarbonsäuren mittels einer anderen Säure, Extraktion der reinen Cannabinoidcarbonsäuren und nachfolgende thermische oder katalytische Zersetzung, oder durch Zersetzung der Salze von Cannabinoidcarbonsäuren mit primären, sekundären oder tertiären Aminen (aber nicht quaternären Ammoniumsalzen), welche ebenfalls thermisch oder katalytisch unterstützt stattfinden kann.

Die Erfindung umfasst ferner cannabinoidcarbonsäurehaltige Liquids und cannabinoidcarbonsäuresalzehaltige Liquids für medizinische Verdampfer.

Solche Liquids sind bevorzugt gegenüber den mit dem Nachteil einer geringen und stark schwankenden Bioverfügbarkeit behafteten oralen Dronabinolzubereitungen, die daüberhinaus auch eine geringe Stabilität gegenüber Oxydation aufweisen.

### 1. Herstellung und Isolation von Cannabinoidcarbonsäuren

### 1.1 Synthetische Herstellung von Cannabinoidcarbonsäuren

### Methode A:

Cannabinoidcarbonsäuren lassen sich synthetisch darstellen durch Carboxylierung aus Neutralcannabinoiden nach aus der Literatur bekannten Verfahren. Siehe hierzu R. Mechoulam et al.: Chem. Communications, 1969, 343-344. Als Ausgangsstoffe können sowohl natürliche wie auch synthetische Cannabinoide dienen.

### Methode B:

Synthetische Cannabinoidcarbonsäuren lassen sich aufbauen durch säurekatalysierte Terpenylierung von Alkylresorcylsäureestern (6-Alkyl-2,4-dihydroxybenzoate) (5) und nachfolgende Verseifung der Ester wie beschrieben in Crombie et al: J. Chem. Research pp.1301-1345 (1977). Bei dieser Terpenylierung führt die Verwendung der optisch aktiven Verbindungen (6a) und (7a) jeweils zu den natürlichen Stereoisomeren der gewünschten Cannabinoidcarbonsäuren und Cannabinoide.

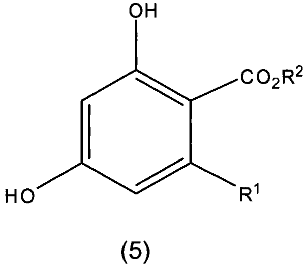

R¹ ist dabei wie oben definiert. R² ist H oder ein geradkettiges oder verzweigtes Alkyl mit bis zu 16 C-Atomen, welches weitere Substituenten wie Phenyl, Hydroxy, Methoxy, Ethoxy, Halogen, Nitril tragen kann.

Bei der Terpenylierung reagieren Verbindungen vom Typ (5) mit Terpenen wie p-Menthadienol (6), Verbenol (7) und Geraniol (8).

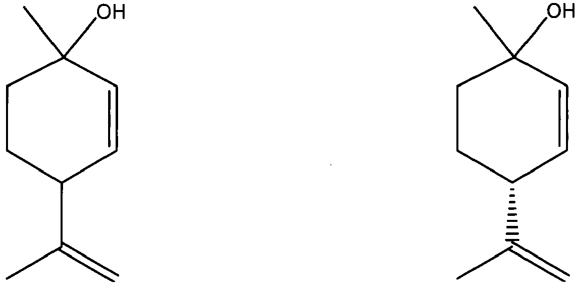

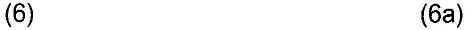

(6a) = (4R)-1-methyl-4-(prop-1-en-2-yl)cyclohex-2-enol

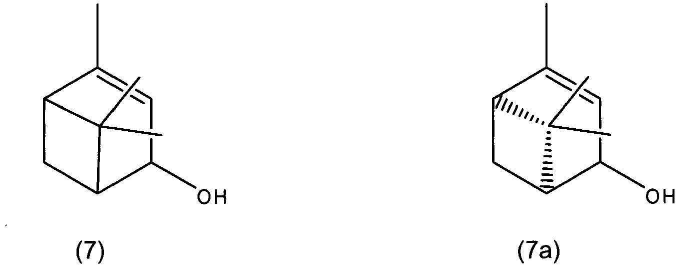

(7a) = (1R,5R)-4,6,6-trimethylbicyclo[3.1.1]hept-3-en-2-ol 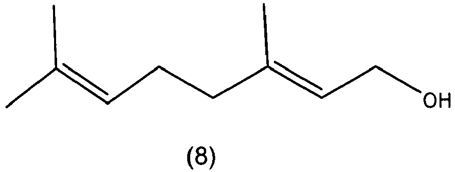 3,7-Dimethylocta-2,6-dien-1-ol

Für den Terpenylierungsschritt geeignete Säuren sind sowohl Brönstedt-Säuren wie auch Lewis-Säuren. Beispiele für geeignete Brönstedt-Säuren sind: Perchlorsäure, Halogenwasserstoffsäuren (HF, HCl, HBr, HI), Schwefelsäure, Hydrogensulfate, Phosphorsäure und ihre sauren Salze, Pyro- und Polyphosphorsäuren, organische Carbon- und Sulfonsäuren mit einem bis zu 30 Kohlenstoffatomen und einer oder mehreren sauren Gruppen, sowie an polymere Träger gebundene saure Gruppen wie z.B. saure Ionenaustauscher und Mischungen der genannten Säuren. Namentlich genannt seien Ameisensäure, Oxalsäure, Trifluoressigsäure, p-Toluolsulfonsäure.

Die Erfindung umfasst durch Bezugnahme vollinhaltlich den Offenbarungsgehalt der europäischen Patentanmeldung EP 2 314 580 (Anmeldung Nr. 10 004 422.1 - 2117), zumindest hinsichtlich der darin beschriebenen Verfahrensweisen der säurekatalysierten Terpenylierung zur Herstellung der Vorläufer der erfindungsgemäßen Salze.

Beispiele für geeignete Lewis-Säuren sind die Kationen von Erdalkali- und Erdmetallen sowie Übergangsmetallen; die Halogenverbindungen und andere dreiwertige Verbindungen von Elementen der dritten Hauptgruppe wie Bortrifluorid und andere Bor-Halogenverbindungen und ihre Komplexe, Aluminiumhalogenide wie wasserfreies Aluminiumchlorid; Salze und Halogenverbindungen von Übergangsmetallen wie Titantetrachlorid, Zinkchlorid, Zinktrifluormethansulfonat; Halogenverbindungen von Elementen der vierten und fünften und sechsten Hauptgruppe wie z. B. Zinntetrachlorid, Phosphortrichlorid, Phosphorpentachlorid, Phosphoroxychlorid, Antimonpentafluorid, Thionylchlorid, Sulfurylchlorid, allein oder in Mischung mit anderen Lewis- oder Brönstedt-Säuren, sowie an polymere Gerüste gebundene positive Zentren wie Montmorillonit.

Weitere geeignete Reagenzien zur Durchführung der Kondensation sind die Acetale von N,N-Dimethylformamid wie z.B. N,N-Dimethylformamid-dineopentylacetal und andere wasserabspaltende Reagenzien, zum Beispiel solche, wie sie für die Bildung von Amiden und Peptiden Verwendung finden wie z. B. PPAA (T3P = Propanphosphonsäureanhydrid).

Diese Reagenzien können als solche zur Reaktionsmischung dazugegeben werden oder auf ein Trägermaterial wie z.B. Aluminiumoxid aufgebracht sein.

Geeignete Lösungsmittel zur Durchführung des Terpenylierungsschrittes sind Wasser, mit Wasser nicht mischbare oder mit Wasser mischbare Lösungsmittel wie z. B. Kohlenwasserstoffe mit bis zu 30 Kohlenstoffatomen, halogenierte Kohlenwasserstoffe mit bis zu 20 C-Atomen wie z.B. Dichlormethan oder Chloroform, Ether wie z. B. 2-Methyltetrahydrofuran, Alkohole, Carbonsäuren mit bis zu 16 C-Atomen, Amide mit bis zu 20 C-Atomen, Ester mit bis zu 60 C-Atomen, Kohlendioxid, Schwefeldioxid, Wasser, Wasser mit einem Phasentransferkatalysator, die sauren Katalysatoren selbst, sowie Mischungen der genannten Lösungsmittel untereinander.

Zur Durchführung versetzt man dabei ein - in der Regel - äquimolares Gemisch aus 6-Alkyl-2,4-dihydroxybenzoesäuren (5) und dem entsprechenden Terpen in einem der genannten Lösungsmittel mit einer katalytischen bis ca. äquimolaren Menge an Säure und rührt bei einer Temperatur zwischen minus 40°C und plus 120°C bis die Umsetzung den erwünschten Grad erreicht hat (Kontrolle mittels Dünnschichtchromatographie oder HPLC).

Anschließend neutralisiert man die Säure mit einer wässrigen Base wie wässrigem Natriumhydrogencarbonat, trennt die organische Phase ab und dampft ein. Man erhält so Cannabinoidcarbonsäureester, welche wie bei Crombie et al. beschrieben zu den entsprechenden Cannabinoidcarbonsäuren (Cannabinoidcarbonsäuren) verseift werden können.

### Methode C:

Die Cannabinoidcarbonsäuren (II), (IV) (V) und (VII), sowie deren Homologen lassen sich auch aus 6-Alkyl-2,4-dihydroxybenzoesäuren (5; R² = H) und den entsprechenden Terpenen direkt aufbauen. Dabei finden die gleichen Reagenzien und Lösungsmittel Verwendung wie bei den Estern der 6-Alkyl-2,4-dihydroxybenzoesäuren.

Durch den Aufbau aus unveresterten 6-Alkyl-2,4-dihydroxybenzoesäuren erspart man sich den nachfolgenden Verseifungsschritt.

### 1.2 Extraktion von Cannabinoidcarbonsäuren aus natürlichem Pflanzenmaterial von Cannabis sativa oder aus Zellkulturen

Die oberirdisch wachsenden Teile von Cannabis sativa (Hanf), sowie Zellkulturen dieser Art enthalten die Cannabinoidcarbonsäuren (II), (IV), (V) und (VII), sowie weitere Cannabinoidcarbonsäuren und niedrige Homologe der genannten in nutzbaren Mengen. Vorteilhaft lässt sich auch ein harzreicher Extrakt aus Pflanzenteilen, wie er z.B. durch das "Ice-o-later" -Verfahren oder durch Aussieben der Harzdrüsen gewonnen werden kann, zur weiteren Anreicherung der Cannabinoidcarbonsäuren einsetzen.

Durch Extraktion mit einem geeigneten Lösungsmittel lässt sich daraus ein Cannabinoidcarbonsäuren-reicher Extrakt gewinnen, in welchem je nach Kultursorte von Cannabis sativa vorwiegend eine der genannten Cannabinoidcarbonsäuren überwiegt.

Dazu bringt man die nach Bedarf getrockneten Pflanzenteile oder Zellkulturen mit einem geeigneten Lösungsmittel in Kontakt und "wäscht" so die überwiegend an der Außenseite der Zelle gespeicherten Cannabinoidcarbonsäuren ab bzw. extrahiert die Zellkulturen.

Zweckmäßigerweise reichert man die erwünschten Cannabinoidcarbonsäuren im Lösungsmittel an, indem man bereits Cannabinoidcarbonsäuren-haltigen Extrakt zur Extraktion weiteren Pflanzenmaterials verwendet.

Vorteilhafterweise verwendet man dabei das Gegenstromverfahren, d. h., man kontaktiert bereits den Cannabinoidcarbonsäuren-haltigen Extrakt mit frischem Pflanzenmaterial und frisches Lösungsmittel mit bereits "gewaschenem" Hanf.

Durch vorsichtiges Eindampfen bei niedrigen Temperaturen, vorzugsweise unter 60°C können so die Cannabinoidcarbonsäuren im rohen Extrakt angereichert werden.

Sofern erforderlich arbeitet man hierbei unter verringertem Druck, um den Siedepunkt des Lösungsmittels herabzusetzen.
i) Geeignete, mit Wasser nicht mischbare Lösungsmittel:
   Kohlenwasserstoffe mit bis zu 30 C-Atomen, auch verflüssigte, im Normalzustand gasförmige Kohlenwasserstoffe wie Propan und/oder Butan, Erdöldestillate wie Petrolether Ligroin, Kerosin, Naphtha, halogenierte Kohlenwasserstoffe mit bis zu 12 C-Atomen, Schwefelkohlenstoff, Ester und Ether mit bis zu 16 C-Atomen, Alkohole, Ketone und Nitrile mit mindestens 4 und bis zu 12 C-Atomen, sowie Mischungen der genannten Lösungsmittel.
ii) Geeignete mit Wasser mischbare Lösungsmittel:
   Wasser mit basischen Zusätzen, wie z.B. Ammoniak, Alkylamine, Hydroxylamin, Hydrazin, Wasser mit Detergentien, niedere Alkohole mit bis zu 4 C-Atomen, Acetonitril, Propionitril, Aceton, sowie Mischungen der genannten Lösungsmittel.
iii) ferner Kohlendioxid und verflüssigtes Schwefeldioxid, verflüssigter Ammoniak, verflüssigte Alkylamine, auch mit Zusätzen der unter i) und ii) genannten Lösungsmittel.

Aus mit Wasser nicht mischbaren Lösungsmitteln lassen sich die Cannabinoidcarbonsäuren auch durch Waschen mit wässrigem Alkali von den neutralen Bestandteilen abtrennen. Man kontaktiert dazu einen mit einem der unter i) genannten Lösungsmittel hergestellten Extrakt mit einer wässrigen Ammoniak Rühren oder Schütteln. Danach lässt man die Phasen trennen und trennt die wässrige Phase ab, welche nun die vorgereinigten (von Neutralbestandteilen weitgehend befreiten) Cannabinoidcarbonsäuren in Form ihrer löslichen Salze enthält.

Durch vorsichtiges Ansäuern (Neutralisieren) mit einer Säure lassen sich daraus die extrahierten Cannabinoidcarbonsäuren fällen und gegebenenfalls mit einem der unter i) genannten Lösungsmittel extrahieren.

### Beispiel:

100 g getrocknete (oder ca. 300 g frische) Blütenstände und Blätter von Cannabis sativa (THC-Typ der Sorte "white widow") werden mit 1 l Petrolether bei < 40°C ausgezogen. Anschließend wird von den ungelösten Pflanzenteilen abfiltriert. Dieser erste Extrakt wird mit 0,5 I wässeriger 0,1 molarer Kalilauge ausgerührt, denen zum Oxidationsschutz 5g Natriumsulfit beigesetzt sein können. Dieser zweite, wässerige Extrakt wird abgetrennt und mit einer Lösung von 5 g Citronensäure in 50 ml Wasser versetzt, worauf die Cannabinoidcarbonsäuren ölig ausfallen. Durch Zugabe von 200 ml Petrolether und Rühren wird nun ein dritter Extrakt hergestellt. Abtrennen der organischen Phase und Eindampfen unter vermindertem Druck bei 40°C ergibt 15,7 g eines öligen Rückstands, der zu 80% aus einer Mischung der Δ⁹-THC-Säuren -A und -B besteht.

Aus den mit unter ii) und iii) genannten Lösungsmitteln gewonnen Extrakten lassen sich die rohen Cannabinoidcarbonsäuren durch Eindampfen, vorzugsweise unter 60°C, gewinnen.

Aus mit Wasser mit basischen Zusätzen gewonnenen Extrakten lassen sich die Cannabinoidcarbonsäuren durch vorsichtiges Ansäuern (Neutralisieren) gewinnen und bei Bedarf mit einem der unter i) genannten Lösungsmittel extrahieren.

Auch durch basische Ionenaustauscher können Cannabinoidcarbonsäuren aus Hanfextrakten von nicht-sauren Bestandteilen abgetrennt werden.

### 2. Kristalline Salze von Cannabinoidcarbonsäuren

### 2.1 Fällung kristalliner Salze mit geeigneten Basen

Werden Cannabinoidcarbonsäuren oder Cannabinoidcarbonsäuren-haltige Extrakte in einem geeigneten Lösungsmittel mit einer geeigneten Base zur Reaktion gebracht, so entstehen kristalline Salze, welche abgetrennt werden können.

Geeignete Lösungsmittel sind Alkohole, Ester, Ether, Ketone, Kohlenwasserstoffe, halogenierte Kohlenwasserstoffe und Nitrile mit bis zu 20 C-Atomen.

Geeignete Basen zur Bildung kristalliner Salze sind primäre, sekundäre und tertiäre organische Amine mit bis zu 48 C-Atomen wie z. B. Dicyclohexylamin und Ammoniak.

Weitere geeignete organische Basen sind pharmazeutische Wirkstoffe mit mindestens einem basischen Stickstoffatom im Molekül, wie z.B. Morphin, Hydromorphon (Palladon®), Buprenorphin, etc.

### Durchführung:

Man gibt unter Rühren eine der erwarteten Menge an erwünschten Cannabinoidcarbonsäuren äquivalente Menge an Base zu der Lösung der Cannabinoidcarbonsäuren in einem geeigneten Lösungsmittel. Vorzugsweise arbeitet man hierbei mit ca. 2%igen bis ca. 20%igen Lösungen von Cannabinoidcarbonsäuren. Man lässt über Nacht auskristallisieren und vervollständigt bei Bedarf die Kristallisation durch Abkühlen auf ca. -10°C. Anschließend wird der ausgefallene Kristallbrei abgesaugt/abzentrifugiert und mit einer geringen Menge des gleichen Lösungsmittels, wie es zur Fällung verwendet wurde, nachgewaschen. Anschließend lässt man bei < 40°C trocknen. Das Salz kann durch Suspendieren in einem der oben genannten Lösungsmittel, Digerieren und Absaugen vom Ungelösten gereinigt werden. Es kann auch aus einem geeigneten Lösungsmittel, vorzugsweise einem niederen Alkohol, Nitril, Keton, Ester oder Ether mit bis zu 4 C-Atomen umkristallisiert werden.

### Beispiel:

15,7 g eines ca. 80%igen Gemisches an Δ⁹-THC-Säuren -A und -B werden unter Rühren in 150 ml Isopropanol gelöst und 8,0 g Dicyclohexylamin unter Rühren zugefügt. 24 h Rühren bei 0°C produziert einen dicken weißen Niederschlag der Dicyclohexylaminsalze der Cannabinoidcarbonsäuren. Nach Absaugen, Waschen mit 50 ml kaltem Isopropanol und Trocknen werden 18,7 g Dicyclohexylaminsalz der Δ⁹-THC-Säuren mit einem Gehalt von 91% erhalten.

**Fällung des Dicyclohexylaminsalzes der CBDS aus einem isopropanolischen Faserhanfextrakt:**

### Beispiel:

2 kg bei unter 30°C getrocknete Blütenstände von Faserhanf (z. B. der Sorte Fedora 19, es sind aber auch andere möglich) werden mit kaltem (< 15°C) Isopropanol portionsweise nach dem Gegenstromverfahren extrahiert. Der gewonnene Extrakt wird unter vermindertem Druck bei max. 40°C auf ein Volumen von ca. 400 ml eingedampft. Dieses Konzentrat wird 12 g Dicyclohexylamin versetzt und 24 h bei 0°C gerührt. Es kristallisiert das Dicyclohexylaminsalz der Cannabidiolsäure, welches abgesaugt wird. Nachwaschen mit 80 ml eiskaltem Isopropanol und trocknen im Vakuum ergibt 22,4 g cremefarbenes Salz.

### 2.2 Fällung von Salzen durch Zugabe eines geeigneten Kations zu einer Lösung von Cannabinoidcarbonsäuren

Zu einer Lösung von Cannabinoidcarbonsäuren in einem geeigneten Lösungsmittel wird eine äquivalente Menge einer Base gegeben, welche in dem verwendeten Lösungsmittel mit den entsprechenden Cannabinoidcarbonsäuren amorphe oder lösliche Salze bildet, wie z. B. Ammoniak. Anschließend wird in die Lösung der Cannabinoidcarbonsäuren-Salze die Lösung eines geeigneten primären, sekundären, tertiären oder quaternären Ammoniumsalzes oder eines Metallsalzes der ersten, zweiten, dritten, vierten oder fünften Hauptgruppe, eines Lanthanidenmetalls oder eines Übergangsmetalls wie z. B. Silber in einem geeignetem Lösungsmittel gegeben. Geeignete Lösungsmittel sind die unter 2.1 genannten.

Geeignete primäre, sekundäre, tertiäre oder quaternäre Ammoniumsalze sind organische Ammoniumsalze mit einem bis zu 48 C-Atomen, welche weitere funktionelle organische Gruppen tragen können.

Das Lösungsmittel und das Ammoniumsalz oder Metallsalz sind so gewählt, dass das jeweilige Kation mit den Anionen der Cannabinoidcarbonsäuren in dem entsprechenden Lösungsmittel einen schwerlöslichen Niederschlag bildet.

Es fällt das entsprechende schwerlösliche Cannabinoidcarbonsäuren-Salz aus, das durch Absaugen oder Zentrifugieren isoliert wird.

### 2.3 Umkristallisation von Cannabinoidcarbonsäuren-Salzen

Da die Cannabinoidcarbonsäuren-Salze gegenüber Decarboxylierung stabiler sind als die freien Säuren, kann man die so gefällten Salze durch Umkristallisation reinigen. Zur Umkristallisation können die gleichen Lösungsmittel wie zur Fällung dienen. Vorzugsweise kristallisiert man aus einem niederen Alkohol, Keton, Nitril, Ester oder Ether mit bis zu 8 C-Atomen um. Der Zusatz einer katalytischen Menge Komplexbildner für Metallkationen, wie z. B. EDTA-Natrium oder eines Kronenethers, kann die Stabilität der Cannabinoidcarbonsäuren-Salze während des Umkristallisiervorgangs erhöhen. Dieses Reinigungsverfahren hat gegenüber den herkömmlichen bei Cannabinoiden verwendeten Verfahren wie der Chromatographie den Vorteil einer wesentlich einfacheren Durchführung.

### Beispiel:

18,7 g Dicyclohexylaminsalz von Δ⁹-THCS-A und -B mit einem Anteil von 91% Δ⁹-THC-Säuren im Cannabinoidanteil werden in 150 ml siedendem absoluten Ethanol unter Rühren gelöst und sofort, nachdem Lösung eingetreten ist, abgekühlt. Zum Auskristallisieren über Nacht bei 0°C rühren. Den gebildeten, weißen Niederschlag absaugen und mit 50 ml kaltem absoluten Ethanol waschen. Ausbeute: 15,5 g reinweißes Salz mit einem Gehalt von > 97% an Δ⁹-THC-Säuren (-A und -B) im Cannabinoidanteil.

Dieses Produkt ist von ausreichender Reinheit, um nach Decarboxylierung pharmazeutisch verwendbares Dronabinol nach DAC 2003 zu liefern.

Eine weitere Anwendung der so hergestellten Cannabinoidcarbonsäuren-Salze besteht als stabile Zusätze zu dermatologischen Produkten. Hierfür werden vorzugsweise Salze von Cannabinoidcarbonsäuren eingesetzt, welche mit toxikologisch am Menschen oder am Tier unbedenklichen oder therapeutisch wirksamen Basen gebildet wurden. Diese Salze müssen nicht kristallin sein, sondern können auch in amorpher Form eingesetzt und den dermatologischen Produkten beigefügt werden. Sie zeichnen sich durch eine erhöhte Stabilität (Lagerfähigkeit) gegenüber freien Cannabinoidcarbonsäuren aus. Eine weitere Stabilisierung kann durch Zugabe von Metallkomplexbildnern wie z. B. EDTA-Natrium erzielt werden, welche Metallionen komplexieren, die die Decarboxylierung von Cannabinoidcarbonsäure-Salzen katalysieren.

### 3. Gewinnung reiner Cannabinoide aus Salzen von Cannabinoidcarbonsäuren

### 3.1 Herstellung reiner Cannabinoide aus Cannabinoidcarbonsäuren-Salzen durch Austreiben der freien Cannabinoidcarbonsäuren

Durch Zugabe einer Säure zu Cannabinoidcarbonsäuren-Salzen werden die Carboxylat-Anionen reprotoniert und die freien lipophilen Säuren lassen sich mit einem geeigneten Lösungsmittel extrahieren. Geeignete Lösungsmittel sind dabei die unter 1.2.i) genannten.

Geeignete Säuren sind wasserlösliche Brönstedtsäuren mit einem pKₐ unterhalb von 7, sowie Kohlensäure (erzeugt z. B. durch Einleiten von CO₂ in Wasser).

Vorzugsweise werden leicht flüchtige Lösungsmittel eingesetzt, welche einen Siedepunkt unter 160°C bei Normaldruck besitzen. Nach Abdestillieren des Lösungsmittels, vorzugsweise bei niedriger Temperatur wie < 60°C, vorzugsweise < 40°C, bleiben die freien Cannabinoidcarbonsäuren als Rückstand zurück.

### Beispiel:

15,0 g umkristallisiertes Dicyclohexylaminsalz der Tetrahydrocannabinolsäuren -A und -B werden in 200 ml Wasser suspendiert und mit 200 ml Petrolether überschichtet. Nun werden 3,0 g Citronensäure zugefügt und so lange gerührt, bis sich das Cannabinoidcarbonsäuren-Salz vollständig gelöst hat. Die wässrige Phase, welche nun das Citrat des Dicyclohexylamins enthält, wird abgetrennt und verworfen. Die Petroletherphase, welche nun die freien Δ⁹-Tetrahydrocannabinolsäuren enthält, wird nacheinander einmal mit 50 ml 1%iger Citronensäure und dreimal mit je 50 ml Wasser gewaschen. Nach dem Eindampfen der Petroletherphase auf dem Wasserbad bei 40°C unter vermindertem Druck bleiben 9,8 g amorpher Rückstand der beiden stellungsisomeren Δ⁹-Tetrahydro-cannabinolsäuren zurück.

### 3.1.1 Cannabinoide durch thermische Zersetzung (Decarboxylierung) von Cannabinoidcarbonsäuren

Durch Erhitzen auf eine Temperatur > 60°C, vorzugsweise über 100°C, werden die freien Cannabinoidcarbonsäuren decarboxyliert, d.h., sie wandeln sich durch Abgabe von Kohlendioxid in die entsprechenden Neutralcannabinoide um. Vorzugsweise arbeitet man dabei im Vakuum oder unter einer Inertgasatmosphäre, um eine Oxidation der entstehenden Cannabinoide zu verhindern. Wird in einem ausreichenden Vakuum gearbeitet, vorzugsweise unter 0,3 mbar, kann das Produkt bei einer Temperatur von vorzugsweise über 140°C gleich destilliert werden. Wird im Gasstrom erhitzt, können die entstehenden Dämpfe der Neutralcannabinoide in einer Inhalationsapparatur medizinisch verabreicht werden.

### Beispiel:

9,8 g Δ⁹-Tetrahydrocannabinolsäuren (-A und -B) mit einer mittels HPLC festgestellten Reinheit von 97,8% werden 30 min lang im Stickstoffstrom auf 160°C erhitzt. Nachdem die Kohlendioxidentwicklung aufgehört hat, bleiben 8,4 g Dronabinol mit einer Reinheit von 97,6% (HPLC) zurück.

### 3.1.2 Cannabinoide durch katalytische Zersetzung (Decarboxylierung) von Cannabinoidcarbonsäuren

Katalysatoren können die Decarboxylierung von Cannabinoidcarbonsäuren wesentlich beschleunigen, so dass diese praktisch augenblicklich quantitativ abläuft. Dies wurde beobachtet an der rasch einsetzenden Blasen-Bildung (CO₂) in freien Cannabinoidcarbonsäuren an Metalloberflächen wie Stahl. Geeignete Katalysatoren sind Elemente der Übergangsmetalle in fein verteilter Form oder mit aktivierten Oberflächen sowie Ionen von Übergangsmetallen. Umgekehrt kann die Desaktivierung von Oberflächen bzw. der Zusatz von Komplexbildnern dazu benutzt werden, um Cannabinoidcarbonsäuren und ihre Salze zu stabilisieren.

### Beispiel:

In einem medizinischen Inhalator (z. B. "Vulcano") werden 0,05 ml einer 5%igen ethanolischen Lösung von reinen Δ⁹-Tetrahydro-cannabinolsäuren (-A und -B) auf das metallische Drahtnetz des Verdampferteils aufgetragen. Anschließend lässt man 60 Sek. einen 230°C heißen Gasstrom durch das Verdampferteil passieren. Im Ballon (Collectorteil) sammelt sich fein vernebeltes Δ⁹-Tetrahydrocannabinol von pharmazeutischer Reinheit für die Inhalation zu medizinischer Anwendung.

### 3.2 Cannabinoide durch Zersetzung von Cannabinoidcarbonsäuren-Salzen

In der Kälte und bei Raumtemperatur sind die Salze von Cannabinoidcarbonsäuren stabile, über Jahre hinweg unzersetzt lagerbare Substanzen. Kristalline Salze von Cannabinoidcarbonsäuren lassen sich darüber hinaus besonders leicht und effektiv durch Umkristallisation reinigen. Diese Eigenschaften kann man sich zu Nutze machen, um sie als quantitative und qualitative Standards in der analytischen Chemie zu verwenden.

### 3.2.1 Thermische Zersetzung von Cannabinoidcarbonsäuren-Salzen

Die Salze von Cannabinoidcarbonsäuren mit primären, sekundären und tertiären Aminen verfügen über ein mobiles Hydrogeniumion (H⁺) im Kation, welches bei erhöhter Temperatur im spürbarem Gleichgewicht mit dem Carboxylat-Anion der Cannabinoidcarbonsäuren-Komponente steht. Im reprotonierten Zustand sind die Cannabinoidcarbonsäuren leicht einer Decarboxylierung zu den entsprechenden Neutralcannabinoiden zugänglich. Werden also Cannabinoidcarbonsäuren-Salze von Ammoniak, primären, sekundären und tertiären Aminen, Hydrazin, Hydroxylamin, Guanidin sowie deren organischen Derivaten stark erhitzt, entweicht leicht Kohlendioxid und es entstehen Mischungen aus den freien Aminen und den Neutralcannabinoiden. Diese liegen bei erhöhter Temperatur, besonders im Vakuum oder in einem Gasstrom in Dampfform vor und können zum Beispiel zur Inhalation benutzt werden.

Eine andere Anwendung liegt in der Verwendung der in der Kälte und bei Raumtemperatur stabilen Salze von Cannabinoidcarbonsäuren als Referenzsubstanzen und Standards für die Gaschromatographie. Dies ist besonders vorteilhaft in den Fällen, in denen die entstehenden Cannabinoide instabile, oxidationsempfindliche Substanzen sind, wie zum Beispiel beim Δ⁹-Tetrahydrocannabinol.

Man injiziert in diesen Fällen eine Lösung eines stabilen Salzes wie z. B. dem Dicyclohexylaminsalzes der Δ⁹-Tetrahydrocannabinolsäure -A oder -B mit einem primären, sekundären oder tertiären Amin in den Injektor des Gaschromatographen. Durch die hohe Temperatur des Injektionsblocks (in der Regel > 230°C) zerfällt das Salz sofort quantitativ in das Neutralcannabinoid (im Falle des Beispiels Δ⁹-Tetrahydrocannabinol), das Amin und CO₂. Durch das Chromatographiesystem wird das Amin (im Falle des Beispiels Dicyclohexylamin) als separater Peak abgetrennt und stört nicht die Quantifizierung des Cannabinoids.

In einem medizinischen Inhalator kann man die Zersetzung von Cannabinoidcarbonsäuren-Salzen in Substanz oder Lösung mit pharmazeutisch aktiven Aminen wie Analgetika, Lokalanästhetika zu einer Kombinationstherapie benutzen, bzw., um den hustenreizenden Effekt von Cannabinoiden wie Dronabinol zu maskieren. Die thermische Zersetzung eines Salzes von Cannabinoidcarbonsäuren mit verdampfbaren pharmakologisch aktiven Aminen führt dazu, dass sowohl das Neutralcannabinoid wie auch die AminKomponente vernebelt werden und in Form eines Aerosols zur Inhalation bereit stehen. Die feine Verteilung der Aerosol-Tröpfchen und die parenterale Verabreichung bedingen eine gegenüber oralen Dosierungsformen wesentlich erhöhte Bioverfügbarkeit.

In der präparativen Chemie stellt die Abtrennung basischer Substanzen wie Aminen von Neutralcannabinoiden kein Problem dar (siehe unter 3.1), so dass sich die Salze von Cannabinoidcarbonsäuren mit primären, sekundären und tertiären Aminen auch hervorragend zur Darstellung reiner Cannabinoide benutzen lassen.

### 3.2.2 Katalytische unterstützte Zersetzung von Cannabinoidcarbonsäuren-Salzen

Auch bei der decarboxylierenden Zersetzung von Cannabinoidcarbonsäure-Salzen wirken die gleichen unter 3.1.2 genannten Substanzen katalytisch wie bei den freien Cannabinoidcarbonsäuren. Man befeuchtet das Salz zur rascheren Umsetzung zu Cannabinoiden mit einer verdünnten (z. B. 0,1%igen) Lösung eines Übergangsmetalls wie zum Beispiel Eisen-[III]-chlorid oder Silbernitrat. Anschließend trocknet man das Salz. Alternativ kontaktiert man das Salz intensiv mit einer kleinen Menge Übergangsmetallen wie z. B. 0,1 Gew.% Stahlpulver, Stahldraht oder 0,01% Silberpulver. Nun erhitzt man das Salz entweder trocken oder zusammen mit einem inerten Wärmeüberträger, wie z. B. einem hochsiedendem Kohlenwasserstoff. Nachdem man das Salz z. B. durch Erhitzen unter Inertgas auf 180°C für 30 min decarboxyliert hat, verfährt man wie unter 3.1 beschrieben, um das Cannabinoid von dem Amin abzutrennen.

Es ist für den Fachmann klar, dass die in dieser Anmeldung beschriebenen Verfahren nicht nur für die explizit genannten Verbindungen, sondern für alle Homologen, deren Stereoisomere sowie für Mischungen daraus (z.B. Racemate) anwendbar sind.

## Patentansprüche

1. Verbindung, ausgewählt aus der Gruppe umfassend alle Stereoisomere sowie deren Mischungen der Verbindungen der allgemeinen Formeln (1) - (4) 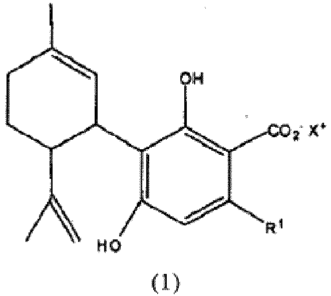 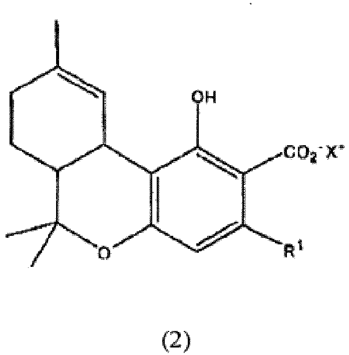 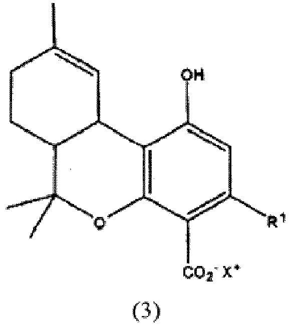 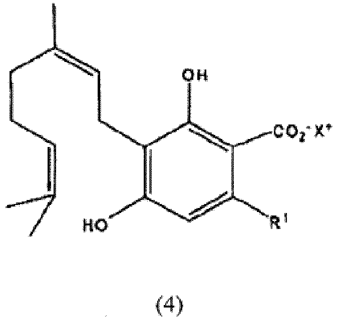 wobei R¹ ein geradkettiger, verzweigter oder zyklischer Kohlenwasserstoffrest mit bis zu 12 C-Atomen ist, und X⁺ ausgewählt ist aus der Gruppe umfassend NH₄⁺, primären, sekundären, tertiären oder quarternären organischen Ammoniumionen mit bis zu 48 C-Atomen, welche noch weitere funktionelle Gruppen tragen können, Hydrazinium-Ion (N₂H₅⁺), Hydroxylammonium-Ion (NH₃OH⁺), Guanidinium-Ion (CN₃H₆⁺), und organischen Derivaten von (N₂H₅⁺), (NH₃OH⁺) und (CN₃H₆⁺), N,N-Dicyclohexylamin-H⁺ oder N,N-Dicyclohexyl-N-ethylamin-H⁺ oder das Hydrogenium-Kation eines pharmazeutischen Wirkstoffes mit mindestens einem basischen Stickstoffatom, insbesondere Morphin, Hydromorphon oder Methadon oder eines Isomeren davon.

2. Verfahren zur Herstellung von Verbindungen ausgewählt aus der Gruppe umfassend alle Stereoisomere sowie deren Mischungen der Verbindungen der allgemeinen Formeln (1) - (4) 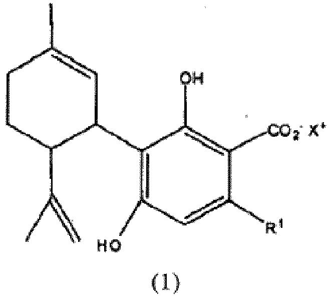 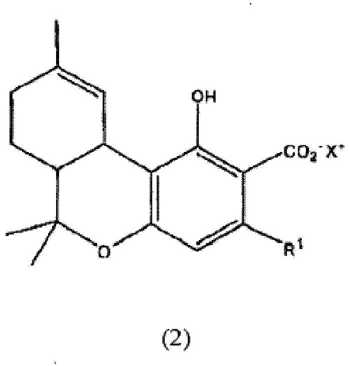 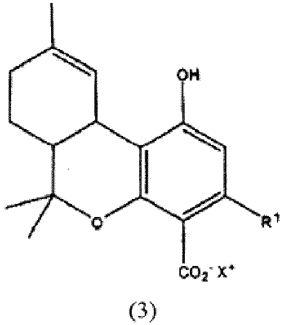 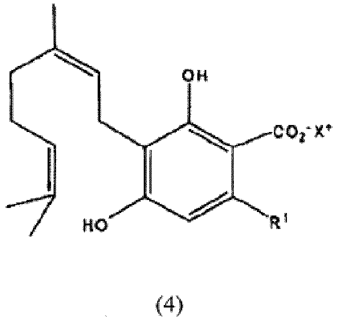
wobei R¹ ein geradkettiger, verzweigter oder zyklischer Kohlenwasserstoffrest mit bis zu 12 C-Atomen ist, und
X⁺ is ausgewählt ist aus der Gruppe umfassend NH₄⁺, primären, sekundären, tertiären oder quarternären organischen Ammoniumionen mit bis zu 48 C-Atomen, welche noch weitere funktionelle Gruppen tragen können, Hydrazinium-Ion (N₂H₅⁺), Hydroxylammonium-Ion (NH₃OH⁺), Guanidinium-Ion (CN₃H₆⁺), und organischen Derivaten von (N₂H₅⁺), (NH₃OH⁺) und (CN₃H₆⁺), N,N-Dicyclohexylamin-H⁺ oder N,N-Dicyclohexyl-N-ethylamin-H⁺ oder das Hydrogenium-Kation eines pharmazeutischen Wirkstoffes mit mindestens einem basischen Stickstoffatom, insbesondere Morphin, Hydromorphon oder Methadon oder eines Isomeren davon, **gekennzeichnet durch** die Schritte
(i) Herstellen von synthetischen Cannabinoidcarbonsäuren in einer chemischen Reaktion oder Extrahierung natürlicher Cannabinoidcarbonsäuren aus Pflanzenmaterial oder Zellkulturen von Cannabis sativa; und
(ii) Versetzen der so hergestellten Cannabinoidcarbonsäuren oder Cannabinoidcarbonsäuren-reichen Extrakte in einem geeigneten Lösungsmittel mit einer geeigneten anorganischen Base, einer geeigneten organischen Base und/oder einem geeigneten anorganischen oder organischen Salz, so dass die schwerlöslichen Salze der Cannabinoidcarbonsäuren gemäß Anspruch 1 ausfallen.

3. Verfahren nach Anspruch 2, **dadurch gekennzeichnet, dass** die chemische Reaktion eine Carboxylierung von natürlichen oder synthetischen Neutralcannabinoiden darstellt.

4. Verfahren nach Anspruch 3, **dadurch gekennzeichnet, dass** eine säurekatalysierte Terpenylierung von ungeschlitzten oder geschlitzten Alkylresorcylsäuren 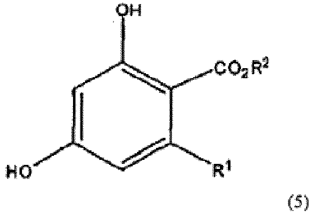 und anschließender Verseifung darstellt, wobei R¹ ein geradkettiger, verzweigter oder zyklischer Kohlenwasserstoffrest mit bis zu 12 C-Atomen ist und R² ein geradkettiges oder verzweigtes Alkyl mit bis zu 16 C-Atomen ist, welches weitere Substituenten wie Phenyl, Hydroxy, Methoxy, Ethoxy, Halogen oder Nitril tragen kann.

5. Verfahren nach Anspruch 2, **dadurch gekennzeichnet, dass** die Extraktion natürlicher Cannabinoidcarbonsäuren mittels eines geeigneten Lösungsmittels bestehend aus der Gruppe bestehend aus Kohlenwasserstoffen mit bis zu 30 C-Atomen; Erdöldestillaten, insbesondere Petrolether, Ligroin, Kerosin, Naphtha; halogenierten Kohlenwasserstoffen mit bis zu 12 C-Atomen, Schwefelkohlenstoff, Ester und Ether mit bis zu 16 C-Atomen, Alkoholen, Ketonen und Nitrilen mit mindestens 4 und bis zu 12 C-Atomen, sowie Mischungen der genannten Lösungsmittel; Wasser mit basischen Zusätzen, Wasser mit Detergentien, niederen Alkoholen mit bis zu 4 C-Atomen, Acetonitril, Propionitril, Aceton, sowie Mischungen der genannten Lösungsmittel; Kohlendioxid und verflüssigtes Schwefeldioxid, auch mit Zusätzen der vorgenannten Lösungsmittel, vorzugsweise im Gegenstromverfahren und anschließendem Eindampfen, vorzugsweise bei Temperaturen unter 60°C vorgenommen wird.

6. Verfahren nach Anspruch 5, **dadurch gekennzeichnet, dass** zur Herstellung der Cannabinoidcarbonsäurensalze das Lösungsmittel ausgewählt ist aus der Gruppe bestehend aus Wasser, Alkoholen, Estern, Ethern, Ketonen, Kohlenwasserstoffen, halogenierten Kohlenwasserstoffen und Nitrilen mit bis zu 20 C-Atomen.

7. Verfahren nach Anspruch 2, **dadurch gekennzeichnet, dass** die anorganischen oder organischen Basen ausgewählt sind aus der Gruppe bestehend aus primären, sekundären und tertiären organischen Aminen mit bis zu 48 C-Atomen, welche noch weitere funktionelle Gruppen tragen können, insbesondere Dicyclohexylamin, Ammoniak, Hydrazin, Hydroxylamin, Guanidin sowie organischen Derivaten von Hydrazin, Hydroxylamin und Guanidin, welche noch weitere funktionelle Gruppen tragen können, Oxiden, Alkoxiden, Hydroxiden, Carbonaten, Hydrogencarbonaten, Caboxylaten sowie anderen basischen Salzen von Elementen der ersten, zweiten und dritten Hauptgruppe des Periodensystems sowie von Zinn, Blei und Wismut und Übergangselementen, wie insbesondere Silber, sowie Ammoniak, entweder allein oder in Mischung.

8. Verfahren nach Anspruch 7, **dadurch gekennzeichnet, dass** die anorganische Base komplexiert ist, um die Löslichkeit zu erhöhen.

9. Verfahren nach Anspruch 2, **dadurch gekennzeichnet, dass** das organische Salz ausgewählt ist aus der Gruppe bestehend aus NH₄⁺, primären, sekundären, tertiären oder quarternären Ammoniumsalzen, insbesondere organischen Ammoniumsalzen mit einem bis zu 48 C-Atomen, welche noch weitere funktionelle Gruppen tragen können, organischen Derivaten von Hydraziniumsalzen, Hydoxylammoniumsalzen und Guanidiniumsalzen, welche noch weitere funktionelle Gruppen tragen können.

10. Verfahren zur Herstellung reiner Cannabinoide aus den Verbindungen nach Anspruch 1 durch Zugabe geeigneter wasserlöslicher Säuren in einem geeigneten Lösungsmittel, vorzugsweise wasserlöslicher Bronsted-Säure mit einem pKa unterhalb von 7 sowie Kohlensäure.

11. Verfahren zur Herstellung reiner Cannabinoide aus den Verbindungen nach Anspruch 1, **dadurch gekennzeichnet, dass** die Verbindungen thermisch zersetzt werden.

12. Verfahren nach Anspruch 11, **dadurch gekennzeichnet, dass** Cannabinoidcarbonsäuren oder ihre Salze zur Beschleunigung der Decarboxylierung mit katalytischen Substanzen kontaktiert werden, insbesondere oberflächenaktiven Übergangsmetallen oder Lösungen von Übergangsmetallen, wie z.B. Eisen-(III)-chlorid oder Silbernitrat.

13. Verwendung von Verbindungen nach Anspruch 1 als Substanz oder deren jeweiligen Lösungen in einem Gerät, das thermisch oder mit katalytischer Unterstützung diese zu Neutralcannabinoiden zersetzt.

14. Verwendung der nach Anspruch 13 erzeugten Neutralcannabinoide als Aerosole in einem Gerät zur Inhalation.

15. Verbindungen nach Anspruch 1 zur Verwendung in der Dermatologie.

## Claims

1. A compound selected from the group comprising all stereoisomers and their mixtures of the compounds of the general formulas (1) - (4) 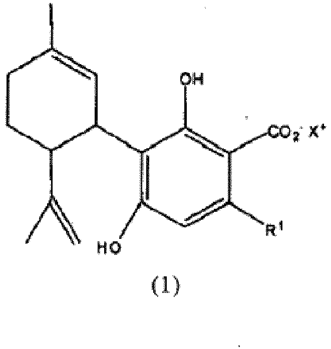 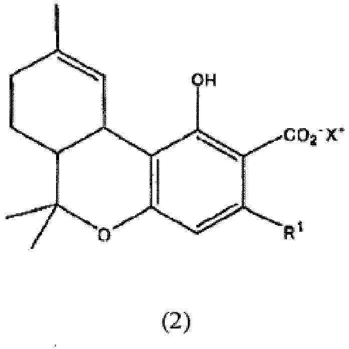 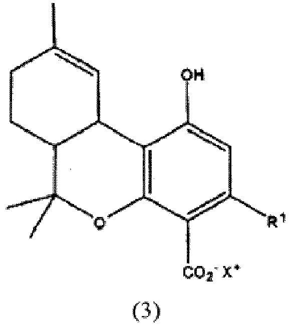 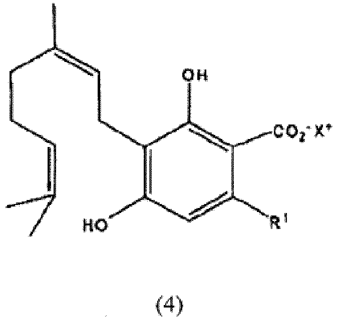 where R¹ is a straight-chain, branched or cyclic hydrocarbon residue with up to 12 carbon atoms, and X⁺ is selected from the group comprising NH₄⁺, primary, secondary, tertiary or quaternary organic ammonium ions with up to 48 carbon atoms, which can also carry other functional groups, hydrazinium ion (N₂H₅⁺), hydroxylammonium ion (NH₃OH⁺), guanidinium ion (CN₃H₆⁺), and organic derivatives of (N₂H₅⁺), (NH₃OH⁺) and (CN₃H₆⁺), N,N-dicyclohexylamine-H⁺ or N,N-dicyclohexyl-N-ethylamine-H⁺ or the hydrogenium cation of an active pharmaceutical ingredient with at least one basic nitrogen atom, in particular morphine, hydromorphone or methadone or an isomer thereof.

2. A method for the manufacture of compounds selected from the group comprising all stereoisomers and their mixtures of the compounds of the general formulas (1) - (4) 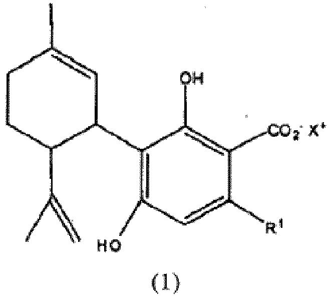 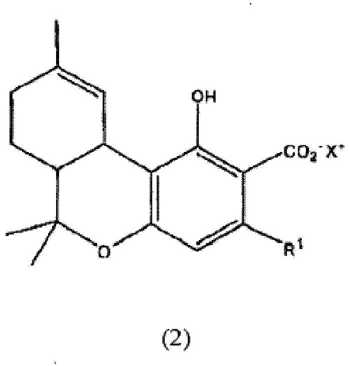 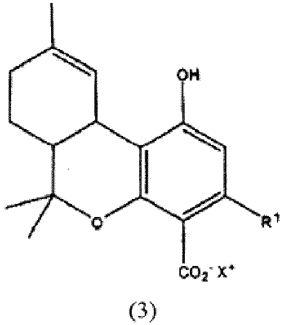 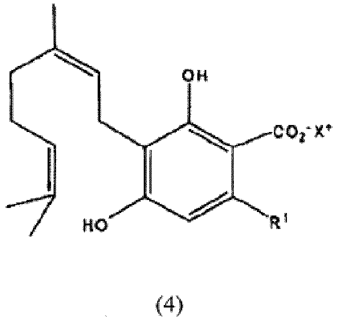
where R¹ is a straight-chain, branched or cyclic hydrocarbon residue with up to 12 carbon atoms, and
X⁺ is selected from the group comprising NH₄⁺, primary, secondary, tertiary or quaternary organic ammonium ions with up to 48 carbon atoms, which can also carry other functional groups, hydrazinium ion (N₂H₅⁺), hydroxylammonium ion (NH₃OH⁺), guanidinium ion (CN₃H₆⁺), and organic derivatives of (N₂H₅⁺), (NH₃OH⁺) and (CN₃H₆⁺), N,N-dicyclohexylamine-H⁺ or N,N-dicyclohexyl-N-ethylamine-H⁺ or the hydrogenium cation of an active pharmaceutical ingredient with at least one basic nitrogen atom, in particular morphine, hydromorphone or methadone or an isomer thereof, **characterized by** the steps
(i) manufacture of synthetic cannabinoid carboxylic acids in a chemical reaction or extraction of natural cannabinoid carboxylic acids from plant material or cell cultures of Cannabis sativa; and
(ii) placing the cannabinoid carboxylic acids or extracts rich in cannabinoid carboxylic acids in a suitable solvent with a suitable inorganic base, a suitable organic base and/or a suitable inorganic or organic salt, so that the sparingly soluble salts of the cannabinoid carboxylic acids according to claim 1 precipitate.

3. The method according to claim 2, **characterized in that** the chemical reaction is a carboxylation of natural or synthetic neutral cannabinoids.

4. The method according to claim 3, **characterized in that** an acid-catalyzed terpenylation of unslotted or slotted alkylresorcylic acids represents 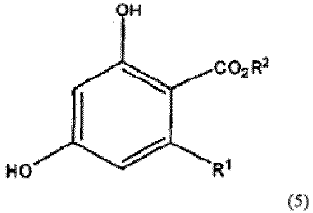 and a subsequent saponification, where R¹ is a straight-chain, branched or cyclic hydrocarbon residue with up to 12 carbon atoms, and R² is a straight-chain or branched alkyl having up to 16 carbon atoms, which can carry further substituents such as phenyl, hydroxy, methoxy, ethoxy, halogen or nitrile.

5. The method according to claim 2, **characterized in that** the extraction of natural cannabinoid carboxylic acids is made by means of a suitable solvent consisting of the group consisting of hydrocarbons with up to 30 carbon atoms; petroleum distillates, in particular petroleum ether, ligroin, kerosene, naphtha; halogenated hydrocarbons with up to 12 carbon atoms, carbon disulfide, esters and ethers with up to 16 carbon atoms, alcohols, ketones and nitriles with at least 4 and up to 12 carbon atoms, as well as mixtures of the solvents mentioned; water with basic additives, water with detergents, lower alcohols with up to 4 carbon atoms, acetonitrile, propionitrile, acetone and mixtures of the solvents mentioned; carbon dioxide and liquefied sulfur dioxide, also with additives of the aforementioned solvents, preferably in the countercurrent process and subsequent evaporation, preferably at temperatures below 60°C.

6. The method according to claim 5, **characterized in that** for the preparation of the cannabinoid carboxylic acid salts the solvent is selected from the group consisting of water, alcohols, esters, ethers, ketones, hydrocarbons, halogenated hydrocarbons and nitriles with up to 20 carbon atoms.

7. The method according to claim 2, **characterized in that** the inorganic or organic bases are selected from the group consisting of primary, secondary and tertiary organic amines having up to 48 carbon atoms, which can also carry further functional groups, in particular dicyclohexylamine, ammonia, hydrazine, hydroxylamine, guanidine and organic derivatives of hydrazine, hydroxylamine and guanidine, which can carry further functional groups, oxides, alkoxides, hydroxides, carbonates, bicarbonates, caboxylates and other basic salts of elements of the first, second and third main group of the periodic table and of tin, lead and bismuth and transition elements, such as in particular silver, and ammonia, either alone or in a mixture.

8. The method according to claim 7, **characterized in that** the inorganic base is complexed to increase the solubility.

9. The method according to claim 2, **characterized in that** the organic salt is selected from the group consisting of NH₄⁺, primary, secondary, tertiary or quaternary ammonium salts, in particular organic ammonium salts having up to 48 carbon atoms, which can carry further functional groups, organic derivatives of hydrazinium salts, hydroxylammonium salts and guanidinium salts, which can carry further functional groups.

10. A method for the manufacture of pure cannabinoids from the compounds according to claim 1 by adding suitable water-soluble acids in a suitable solvent, preferably water-soluble Bronsted acid with a pKa below 7 and carbonic acid.

11. A method for the manufacture pure cannabinoids from the compounds according to claim 1, **characterized in that** the compounds are thermally decomposed.

12. The method according to claim 11, **characterized in that** cannabinoid carboxylic acids or their salts are contacted with catalytic substances to accelerate decarboxylation, in particular surface-active transition metals or solutions of transition metals, such as ferric chloride or silver nitrate.

13. The use of the compound according to claim 1 as a substance or their respective solutions in a device which decomposes the same thermally or with catalytic support to neutral cannabinoids.

14. The use of the neutral cannabinoids produced according to claim 13 as aerosols in a device for inhalation.

15. The compound according to claim 1 for use in dermatology.

## Revendications

1. Liaison, choisie dans le groupe comprenant tous les stéréo-isomères et leurs mélanges des liaisons des formules générales (1) - (4) 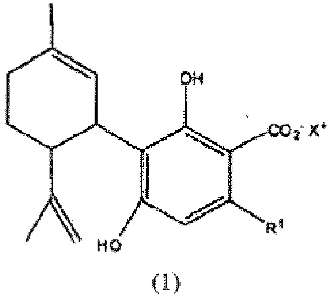 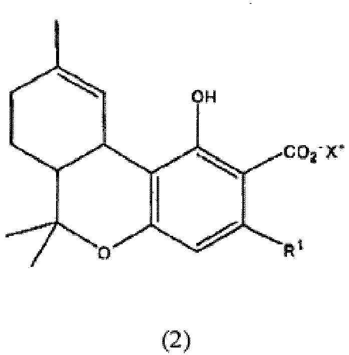 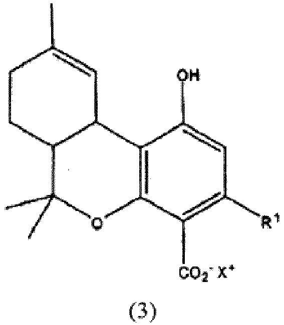 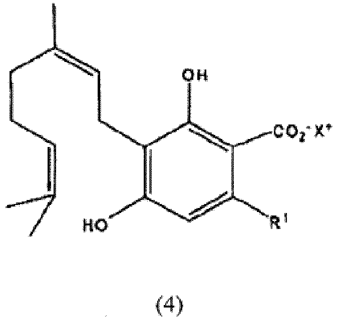 dans laquelle R1 est un résidu d'hydrocarbure en chaîne linéaire, ramifié ou cyclique avec jusqu'à 12 atomes de C, et X+ est choisi dans le groupe comprenant NH4+, des ions d'ammonium organiques primaires, secondaires, tertiaires ou quaternaires avec jusqu'à 48 atomes de C, lesquels peuvent encore porter d'autres groupes fonctionnels, l'ion hydrazinium (N2H5+), l'ion hydroxylammonium (NH3OH+), l'ion guanidinium (CN3H6+), et des dérivés organiques de (N2H5+), (NH3OH+) et (CN3H6+), N,N-dicyclohexylamine-H+ ou N,N-dicyclohexyl-N-éthylamine-H+ ou le cation d'hydrogène d'une substance active pharmaceutique avec au moins un atome d'azote basique, plus particulièrement de la morphine, de l'hydromorphone ou de la méthadone ou d'un isomère de celles-ci.

2. Procédé de fabrication de liaisons choisies dans le groupe comprenant tous les stéréo-isomères et leurs mélanges des liaisons des formules générales (1) - (4) 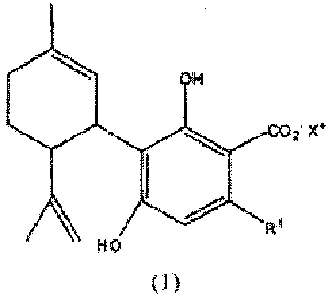 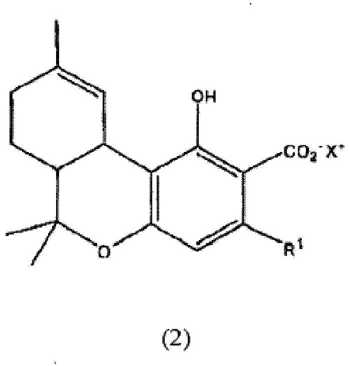 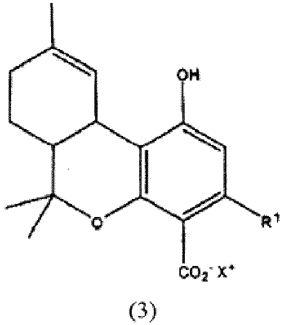 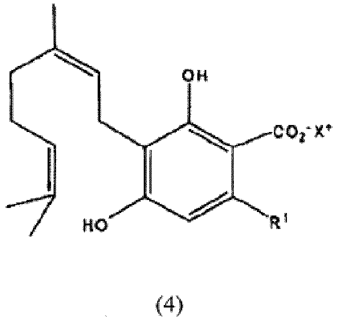 dans lequel R1 est un résidu d'hydrocarbure en chaîne linéaire, ramifié ou cyclique avec jusqu'à 12 atomes de C, et X+ est choisi dans le groupe comprenant NH4+, des ions d'ammonium organiques primaires, secondaires, tertiaires ou quaternaires avec jusqu'à 48 atomes de C, lesquels peuvent encore porter d'autres groupes fonctionnels, l'ion hydrazinium (N2H5+), l'ion hydroxylammonium (NH3OH+), l'ion guanidinium (CN3H6+), et des dérivés organiques de (N2H5+), (NH3OH+) et (CN3H6+), N,N-dicyclohexylamine-H+ ou N,N-dicyclohexyl-N-éthylamine-H+ ou le cation d'hydrogène d'une substance active pharmaceutique avec au moins un atome d'azote basique, plus particulièrement de la morphine, de l'hydromorphone ou de la méthadone ou d'un isomère de celles-ci, **caractérisé par** les étapes de
i.) fabrication d'acides carboxyliques de cannabinoïde synthétiques dans une réaction chimique ou une extraction d'acides carboxyliques de cannabinoïde naturels à partir de matériel végétal ou de cultures cellulaires de cannabis sativa ; et
ii) mélange des acides carboxyliques de cannabinoïde ou des extraits riches en acides carboxyliques de cannabinoïde ainsi fabriqués dans un solvant approprié avec une base inorganique appropriée, une base organique appropriée et / ou un sel inorganique ou organique approprié de sorte que les sels solubles difficilement des acides carboxyliques de cannabinoïde selon la revendication 1 se précipitent.

3. Procédé selon la revendication 2, **caractérisé en ce que** la réaction chimique représente une carboxylation de cannabinoïdes neutres naturels ou synthétiques.

4. Procédé selon la revendication 3, **caractérisé en ce qu'**une terpénylation catalysée par acide d'acides résorcyliques alkyles non protégés ou protégés 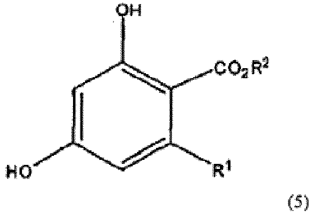 et une saponification consécutive est obtenue, dans lequel R1 est un résidu d'hydrocarbure en chaîne linéaire, ramifié ou cyclique avec jusqu'à 12 atomes de C et R2 est un alkyle en chaîne linéaire ou ramifié avec jusqu'à 16 atomes de C, lequel peut porter d'autres substituants tels que phényle, hydroxy, méthoxy, éthoxy, halogène ou nitrile.

5. Procédé selon la revendication 2, **caractérisé en ce que** l'extraction d'acides carboxyliques de cannabinoïde naturels est effectuée à l'aide d'un solvant approprié composé du groupe composé d'hydrocarbures avec jusqu'à 30 atomes de C ; de distillats du pétrole, plus particulièrement d'éther de pétrole, de ligroïne, de pétrole lampant, de naphta ; d'hydrocarbures halogénés avec jusqu'à 12 atomes de C, de sulfure de carbone, d'ester et d'éther avec jusqu'à 16 atomes de C, d'alcools, de cétones et de nitriles avec au moins 4 et jusqu'à 12 atomes de C, ainsi que de mélanges des solvants cités ; de l'eau avec des additifs basiques, de l'eau avec des détergents, des alcools inférieurs avec jusqu'à 4 atomes de C, de l'acétonitrile, du propionitrile, de l'acétone, ainsi que des mélanges des solvants cités ; du dioxyde de carbone et du dioxyde de soufre liquéfié, également avec des additifs des solvants précités, de préférence dans un procédé à contre-courant et évaporation consécutive, de préférence à des températures inférieures à 60°C.

6. Procédé selon la revendication 5, **caractérisé en ce que** pour fabriquer les sels d'acides carboxyliques de cannabinoïde, le solvant est choisi dans le groupe composé d'eau, d'alcools, d'esters, d'éthers, de cétones, d'hydrocarbures, d'hydrocarbures halogénés et de nitriles avec jusqu'à 20 atomes de C.

7. Procédé selon la revendication 2, **caractérisé en ce que** les bases inorganiques et organiques sont choisies dans le groupe composé d'amines organiques primaires, secondaires et tertiaires avec jusqu'à 48 atomes de C, lesquelles peuvent encore porter d'autres groupes fonctionnels, plus particulièrement la dicyclohéxylamine, l'ammoniac, l'hydrazine, l'hydroxylamine, la guanidine, lesquelles peuvent encore porter d'autres groupes fonctionnels, des oxydes, des alcoolates, des hydroxydes, des carbonates, des hydrogénocarbonates, des carboxylates ainsi que d'autres sels basiques d'éléments du premier, deuxième et troisième groupe principal du système périodique ainsi que d'étain, de plomb et de bismuth et d'éléments de transition comme l'argent notamment, ainsi que d'ammoniaque, soit seule ou en mélange.

8. Procédé selon la revendication 7, **caractérisé en ce que** la base inorganique est complexée afin d'accroître la solubilité.

9. Procédé selon la revendication 2, **caractérisé en ce que** le sel organique est choisi dans le groupe composé de NH4+, de sels d'ammonium primaires, secondaires, tertiaires ou quaternaires, plus particulièrement de sels d'ammonium organiques avec un jusqu'à 48 atomes de C, lesquels peuvent encore porter d'autres groupes fonctionnels, de dérivés organiques de sels d'hydrazine, de sels d'hydroxylammonium et de sels de guanidinium, lesquels peuvent encore porter d'autres groupes fonctionnels.

10. Procédé de fabrication de cannabinoïde pure à partir des liaisons selon la revendication 1 par addition d'acides solubles dans l'eau appropriés dans un solvant approprié, de préférence d'acides de Brönsted solubles dans l'eau avec un pKa inférieur à 7 ainsi que d'acides carboniques.

11. Procédé de fabrication de cannabinoïde pure à partir des liaisons selon la revendication 1, **caractérisé en ce que** les liaisons sont décomposées thermiquement.

12. Procédé selon la revendication 11, **caractérisé en ce que** les acides carboxyliques de cannabinoïde ou leurs sels sont mis en contact avec des substances catalytiques pour accélérer la décarboxylation, plus particulièrement avec des métaux de transition tensio-actifs ou des solutions de métaux de transition comme, par exemple, le chlorure de fer III ou le nitrate d'argent.

13. Utilisation de liaisons selon la revendication 1 comme substance ou leurs solutions respectives dans un appareil qui décompose celles-ci, thermiquement ou avec une assistance catalytique, en cannabinoïdes neutres.

14. Utilisation de cannabinoïdes neutres produits selon la revendication 13 comme aérosols dans un appareil pour l'inhalation.

15. Liaisons selon la revendication 1 destinées à être utilisées en dermatologie.
